# EUROPÄISCHE PATENTSCHRIFT

(11) **EP 2 317 926 B1**
(45) Veröffentlichungstag und Bekanntmachung des Hinweises auf die Patenterteilung: **16.03.2016**
(21) Anmeldenummer: 09781123.6
(22) Anmeldetag: 27.07.2009
(51) Int. Cl.: A61B 6/14, A61B 6/12

(54) **DENTALMEDIZINISCHES ERZEUGNIS ZUR GRÖSSENORDNUNGSGERECHTEN AUSWERTUNG VON RÖNTGENAUFNAHMEN VON ZU DIAGNOSTIZIERENDEN BEREICHEN INNERHALB DER MUNDHÖHLE EINES PATIENTEN SOWIE VERWENDUNG EINES DENTALMEDIZINISCHEN ERZEUGNISSES**
DENTAL PRODUCT FOR MAGNITUDE-CORRECT EVALUATION OF X-RAY IMAGES OF AREAS TO BE DIAGNOSED WITHIN THE ORAL CAVITY OF A PATIENT, AND USE OF A DENTAL PRODUCT
PRODUIT DE MÉDECINE DENTAIRE POUR ÉVALUATION DES ORDRES DE GRANDEUR DANS DES ENREGISTREMENTS RADIOGRAPHIQUES DE PARTIES À DIAGNOSTIQUER À L'INTÉRIEUR DE LA CAVITÉ BUCCALE D'UN PATIENT ET UTILISATION D'UN PRODUIT DE MÉDECINE DENTAIRE

(30) Priorität: 30.07.2008 DE 102008035504
(43) Veröffentlichungstag der Anmeldung: 11.05.2011
(73) Patentinhaber: Middelmann, Heinrich, 85716 Unterschleissheim (DE)
(72) Erfinder: Middelmann, Heinrich, 85716 Unterschleissheim (DE)
(74) Vertreter: Grosse, Felix Christopher
(86) Internationale Anmeldenummer: PCT/EP2009/059661
(87) Internationale Veröffentlichungsnummer: WO 2010/012680

(56) Entgegenhaltungen:
- KR-A- 20050 000 934
- US-A- 5 878 104
- US-A1- 2006 241 406
- US-B1- 6 289 235

## Beschreibung

Die Erfindung betrifft ein dentalmedizinsches Erzeugnis zur größenordnungsgemäßen Auswertung von Röntgenaufnahmen von zu diagnostizierenden Bereichen, insbesondere Zahn- und Kieferknochenbereichen, innerhalb der Mundhöhle eines Patienten.

Zum Hintergrund der Erfindung gehört, dass es bei der Diagnostik im Dentalbereich, wenn üblicherweise Röntgenaufnahmen anzufertigen sind, es sehr schwer ist, die Aufnahmen verzerrungsfrei anzufertigen. Dies ist damit begründet, dass eine parallele Ausrichtung der Röntgenaufnahmenvorrichtung, des aufzunehmenden Objektes, wie eines Zahnes bzw. eines Teilbereiches des Kiefers sowie des Aufnahmemediums in der Regel nicht möglich ist. Ferner ist es meist nicht möglich, die Röntgenstrahlen genau parallel auszurichten. Wird in unmittelbarer Nähe des aufzunehmenden Objektes ein Referenzkörper mit bekannten Abmessungen angeordnet und mit abgebildet, kann ein Maß für die Schräglage zwischen Aufnahmeebene und Objekt erhalten werden. Durch Auswertemittel, die einen Korrekturfaktor heranziehen, der dem Maß der Schräglage entspricht, kann aus einem verzerrten Abbild des aufgenommenen Objektes ein verzerrungsfreies Abbild des aufgenommenen Objektes gewonnen werden. Es hat sich jedoch in Praxis als schwierig erwiesen, ein Referenzobjekt in einer einfachen und sicheren Weise in der Nähe des aufzunehmenden Bereiches anzubringen.

Aus der DE 196 19 924 A1 ist es bekannt, ein Referenzobjekt durch Verklebung am Untersuchungsobjekt anzubringen. Hierbei ist es jedoch nachteilig, dass die Verklebung, zum Beispiel einer Kugel am Untersuchungsobjekt, wie einem Zahn, unnötig viel Vorbereitungsaufwand vor Durchführung der eigentlichen Röntgenaufnahme erforderlich macht und es doch nicht vollkommen ausgeschlossen werden kann, dass die anschließende Entfernung der Kugel vom Zahn Schäden am Zahn hinterlässt. Wird, wie auch noch in der DE 196 19 924 A1 offenbart, eine Kappe mit mindestens einem Referenzobjekt über das z.B. einen Zahn betreffende Untersuchungsobjekt gestülpt, so geht damit auch ein nicht unerheblicher Zeit- und Kostenaufwand einher.

Aus der US 2006/0241406 A1 geht eine Registraturvorrichtung für anatomische Daten hervor. In einem formbaren Material sind röntgenstrahlen-undurchlässige Formkörper eingebracht. Das Material ist in den Mund eines Patienten einführbar.

Durch die US 6,289,235 B1 gehört ein System zur Schaffung dreidimensionaler Bilder durch Synthese von tomographischen Aufnahmen zum Stand der Technik. Das System umfasst einen Beißblock, der in den Mund eines Patienten einführbar ist und an dem ein Referenzelement befestigt ist.

Die KR 10-2005-000934 A zeigt eine Referenzvorrichtung in Form eines Beißblocks auf, in den in regelmäßigen Abständen Metallkugeln eingebracht sind.

Gemäß der US 5,878,104 ist als Vorrichtung eine Kappe vorgesehen, auf der röntgenstrahlen-undurchlässige Referenzkörper angeordnet sind und die auf einem Untersuchungsobjekt, z. B. einem Zahn, platzierbar ist.

Es ist auch bekannt, laborgefertigte individuelle Schablonen oder spezielle Silikonabdruckmassen zu verwenden, in die das mindestens eine Referenzobjekt eingebettet ist.

Auch hierbei ist ein erheblicher Zeitaufwand erforderlich, um die Aufnahme für das mindestens eine Referenzobjekt vor Einsatz beim Patienten vorzubereiten, wobei die damit einhergehenden Kosten erheblich sind. Nach dem Gebrauch der für einen bestimmten Patienten hergestellten Schablonen ist diese nicht wieder zu verwenden und muss entsorgt werden.

Somit ist es Aufgabe der Erfindung, ein alternatives dentalmedizinisches Erzeugnis zur größenordnungsgerechten Auswertung von Röntgenaufnahmen von zu diagnostizierenden Bereichen, insbesondere Zahn- und /oder Kieferknochenbereichen, innerhalb der Mundhöhle eines Patienten bereitzustellen, das einfach- und kostengünstig ist.

Ferner soll eine einfache Verwendung eines dentalmedizinischen Erzeugnisses bereitgestellt werden.

Die Aufgabe wird für ein dentalmedizinisches Erzeugnis zur größenordnungsgerechten Auswertung von Röntgenaufnahmen von zu diagnostizierenden Bereichen, insbesondere Zahn- und /oder Kieferknochen-Bereichen, innerhalb der Mundhöhle eines Patienten, dadurch gelöst, dass das Erzeugnis aufweist: einen Hauptkörper, dessen Gestalt so ist, dass er vom Patienten aufnehmbar ist und in unmittelbarer Nähe des durch Röntgen zu diagnostizierenden Bereiches fixierbar ist, bestehend aus einem röntgenstrahlen-durchlässigem Material, wobei im Innenraum des Hauptkörpers mindestens ein Referenzobjekt aufgenommen ist, das aus röntgenstrahlen-absorbierenden Material besteht. Die Gestalt des Hauptkörpers ist dabei kugelförmig oder länglich und hat einen im Wesentlichen kreisrunden Querschnitt. Das Material des Hauptkörpers ist nachgiebig bzw. weich ist. Die Gestalt des Hauptkörpers ist dabei kugelförmig oder länglich und hat einen im Wesentlichen kreisrunden Querschnitt, wobei der Hauptkörper durch eine Zellstoff- bzw. Watterolle gebildet ist. Es wurde herausgefunden, dass eine im Bereich der Zahnmedizin bekannte Zellstoffrolle bzw. Watterolle sich als einfach zu handhabendes und preiswertes Ausgangsgut für den Hauptkörper darstellt. Der mindestens eine Referenzkörper kann dann in einem geeigneten Schritt der Herstellung der Zellstoff- bzw. Watterolle eingefügt werden.

Hinsichtlich der Wahl des mindestens einen Referenzkörpers bestehen keine grundsätzlichen Einschränkungen. Es kommt nur darauf an, dass der mindestens eine Referenzkörper aus einem formstabilen Material besteht, das röntgenstrahlen-absorbferend bzw. röntgenstrahlendicht ist. Das mindestens eine Referenzobjekt kann ein Stift oder ein Röhrchen oder ein kugelartiger, 3-achssymmetrischer Köper insbesondere eine Kugel sein. Als Material kann insbesondere Stahl oder Titan gewählt werden. Wenn zwei Referenzobjekte zur größenordnungsgerechten Auswertung der Röntgenaufnahme sich als erforderlich erweisen, werden vorzugsweise Kugeln als Referenzobjekte gewählt, die einen bekannten Durchmesser von z. B. 0,5 mm haben und damit ein geeignetes Größenverhältnis zu üblichen Zellstoff- oder Watterollen haben, die bevorzugt zur Bildung des Hauptkörpers eingesetzt werden.

Bei sehr hohen Ansprüchen an die größenardnungsgerechte Auswertung von Röntgenaufnahmen kann es sich als erforderlich erweisen, dass mehr als zwei Referenzobjekte vorgesehen sind, die einen räumlichen Verbund bilden, wobei die Verbundstege zwischen den Referenzobjekten aus nahezu röntgenstrahlen-durchlässigem Material bestehen.

Bei der Verwendung eines dentalmedizinischen Erzeugnisses wird berücksichtigt, dass für die größenordnungsgerechte Auswertung von Röntgenaufnahmen von zu diagnostizierenden Bereichen, insbesondere von Zahn- und / oder Kieferknochen-Bereichen, innerhalb der Mundhöhle eines Patienten, mindestens ein dentalmedizinisches Erzeugnis dem Patienten in den Mund gegeben wird und in der Weise in unmittelbarer Nähe des zu diagnostizierenden Bereiches fixierbar ist, indem der Patient das mindestens eine dentalmedizinische Erzeugnis durch Aufbeißen fixiert und ein beim Röntgen zunächst verzerrtes Abbild des diagnostizierten Bereiches über die bekannte Größe des mindestens einen im Innenraum eines Hauptkörpers des dentalmedizinischen Erzeugnisses aufgenommenen Referenzobjektes aus röntgenstrahlen-dichtem bzw. -absorbierendem Material korrigierend ausgerechnet wird, so dass ein größenordnungsgerechtes Bild bzw. eine größenordnungsgerechte Anzeige entsteht.

Die Erfindung wird nachfolgend anhand von in der Zeichnung mit mehreren Figuren dargestellter Ausführungsbeispiele näher beschrieben.

Es zeigen:
- Fig. 1: eine Röntgendiagnostikeinrichtung, wie sie z. B. bei der Erfindung zum Einsatz gelangt,
- Fig. 2: eine etwas größere Darstellung im Schnitt des erfindungsgemäßen, dentalmedizinischen Erzeugnisses mit einem zentral aufgenommenen Referenzobjekt,
- Fig. 3: eine nicht maßstäbliche Darstellung von einer seitlichen Schnittansicht des erfindungsgemäßen, dentalmedizinischen Erzeugnisses mit einem aufgenommenen Referenzobjekt und daneben mit dargestellter Querschnittsansicht entlang Schnittlinie S-S,
- Fig. 4: eine nicht maßstäbliche Darstellung von einer seitlichen Schnittansicht des erfindungsgemäßen dentalmedizinischen Erzeugnisses mit zwei aufgenommenen Referenzobjekten und daneben mit dargestellter Querschnittsansicht entlang Schnittlinie S'-S'.
- Fig. 5: einen räumlichen Verbund mit mehr als zwei Referenzobjekten, eingebracht in den schematisch dargestellten Hauptkörper, und
- Fig. 6: eine gewöhnliche Einbringung des erfindungsgemäßen dentalmedizinischen Erzeugnisses im Mundraum des Patienten und dessen Fixierung durch Aufbeißen

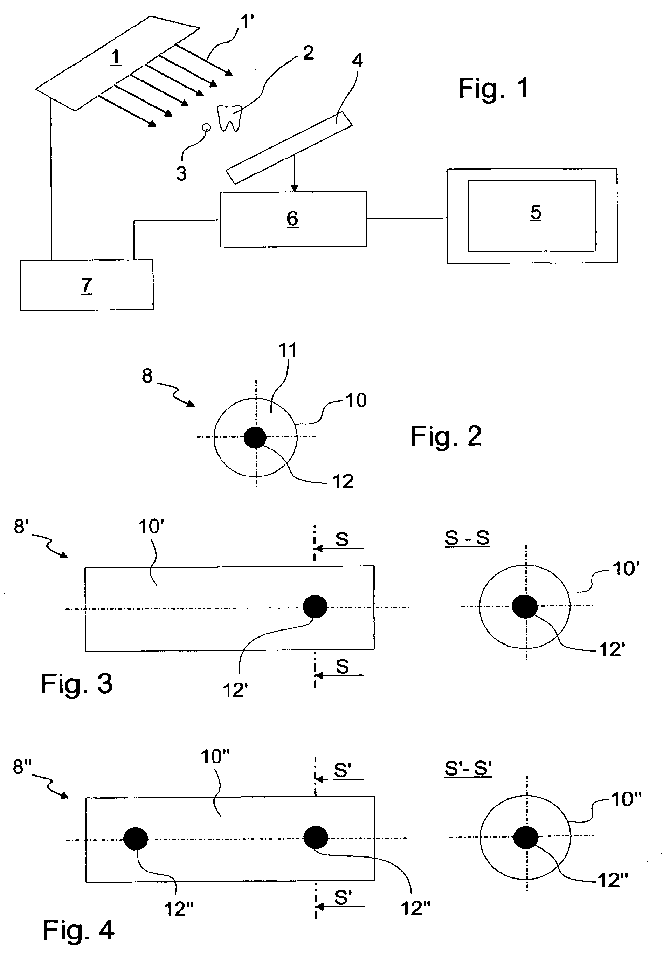

Eine beispielsweise im Zusammenhang mit der Erfindung zum Einsatz gelangende, jedoch nicht zur Erfindung gehörende, Röntgendiagnostikeinrichtung ist in Fig. 1 dargestellt. Ausgehend von einer Röntgenstrahlungsquelle 1 werden austretende Röntgenstrahlen 1' auf einen zu diagnostizierenden Bereich mit zum Beispiel einem zu untersuchenden Objekt 2, wie ein Zahn, gerichtet, um eine Abbildung auf einem Aufnahmemedium 4 zu erhalten. Neben dem zu untersuchenden Objekt wird auch ein allgemeines Referenzobjekt 3 bekannter Dimension aus formstabilem und röntgenstrahlen-absorbierendem Material, wie z. B. eine Stahl- oder Titankugel, auf dem Aufnahmemedium 4 abgebildet. Die auf dem Aufnahmemedium 4 erhaltene Abbildung des zu untersuchenden Objektes 2 weist in aller Regel eine Verzerrung auf. Mithilfe der auch verzerrten Abbildung des Referenzobjektes 3, dessen Originaldimension bekannt ist - bei Abbildung einer Kugel wird anstelle eines Kreises eine Ellipse abgebildet -kann aus dem Verhältnis der Halbachsen der Ellipse ein Maß für die Schräglage von Aufnahmeebene zu Abbildungsebene des Aufnahmemediums gewonnenen werden, so dass zusätzlich mit der bekannten Dimension des Referenzobjektes 3 über eine Auswerteeinheit 6, wie Rechner, die mit dem Aufnahmemedium 4, z. B. in höchster Version einem Halbleiterarray, in Verbindung stehen, aus dem digitalen Abbild von Untersuchungsobjekt 2 und Referenzobjekt 3 entsprechend von durch ein Bedienfeld 7 eingegebenen Vorgaben an einem Bildschirm 5, der mit der Auswerteeinheit 6 in Verbindung steht, eine verzerrungsfreie Darstellung des Untersuchungsobjektes 2, wie eines Zahns, oder ein genaues Abbild eines Knochenaufbaus im Umfeld eines Zahnes, erhalten werden. In der Auswerteeinheit 6 gelangen geeignete Rechenalgorithmen zum Einsatz, die vom Ansatz her alle auf Verhältnisrechnungen, auch oft mit Dreisatz bezeichnet, beruhen. Auf diese Weise kann die Dimension der Knochenstruktur errechnet werden, was für die Einbringung z. B. eines Implantates notwendige Voraussetzung ist.

Mit Fig. 2 ist etwas größer ein Querschnitt des erfindungsgemäßen dentalmedizinischen Erzeugnisses 8 dargestellt. Da das dentalmedizinische Erzeugnis 8 auch eine Kugel sein kann, ist dafür diese Darstellung auch zutreffend. Es ist erkennbar, wie in einem Hauptkörper 10 mit einem Innenraum 11 mindestens ein Referenzobjekt 12 nahezu zentral aufgenommen ist. Für den Patienten ist es angenehm, wenn der Hauptkörper nachgiebig bzw. weich ist. Hierfür ist Zellstoff bzw. Watte äußerst geeignet. Es können jedoch auch andere Materialien verwendet werden, die diese Eigenschaft - nachgiebig / weich - aufweisen. Da der Patient den Hauptkörper durch Aufbeißen über die Dauer des Diagnosevorganges durch Röntgen fixieren soll, ist das Ausmaß der Nachgiebigkeit geeignet zu berücksichtigen, denn das Referenzobjekt soll nicht beschädigt werden oder das Aufbeißen beim Patienten Schmerzen verursachen. Es kommt lediglich darauf an, dass das Referenzobjekt im Bereich der zu untersuchenden bzw. zu vermessenden Stelle des Knochens während der Aufnahme fixiert ist. Zudem ist es erforderlich, dass das Material des Hauptkörpers röntgenstrahlendurchlässig ist.

Mit Fig. 3 ist eine Seitenansicht des erfindungsgemäßen dentalmedizinischen Erzeugnisses dargestellt. Der Hauptkörper 10' hat eine längliche Gestalt und einen im Wesentlichen kreisrunden Querschnitt. Überraschenderweise konnte herausgefunden werden, dass eine im Bereich der Zahnmedizin bekannte Zellstoffrolle bzw. Watterolle sich als hervorragend geeignet für den Hauptkörper anbietet. Das mindestens eine Referenzobjekt kann dann in einem geeigneten Schritt der Herstellung der Zellstoff- bzw. Watterolle eingefügt werden.

Nach Fig. 3 ist im Hauptkörper 10' ist Referenzobjekt 12' im Wesentlichen im Bereich von dessen Längsachse, jedoch nicht mittig zu dessen Längsdimension auf genommen. Hiermit soll einem später erfolgenden Einsatz des dentalmedizinischen Erzeugnisses hinsichtlich besserer Zuordenbarkeit des Referenzobjektes 12' zum zu diagnostizierenden. Bereich Rechnung getragen werden.

Mit Fig. 4 wurde eine Darstellung des erfindungsgemäßen dentalmedizinischen Erzeugnisses mit Hauptkörper 10" und zwei aufgenommenen Referenzobjekten 12" vorgenommen. Durch die zwei Referenzobjekte 12" kann einerseits auch eine bessere Zuordenbarkeit von einem der Referenzobjekte 12" zum durch Röntgen diagnostizierenden Bereich vorgenommen werden. Andererseits ist es möglich, durch zwei Referenzobjekte 12" mehr Referenzinformationen zu erhalten, so dass die Auswerteeinrichtung 6 eine noch weiter verbesserte, größenordnungsgerechte Auswertung vorzunehmen vermag und auf dem Bildschirm 5 anzuzeigen vermag.

Bei dem nach den Fig. 3 und 4 erkennbaren Hauptkörper 10' und 10" hat dieser eine längliche Gestalt. In bevorzugter Ausführung der Erfindung kann dieser durch eine Zellstoff- bzw. Watterolle gebildet werden. Es sind zunächst keine einschränkenden Anforderungen an die Referenzobjekte 12' und 12" vorliegend. Als Referenzobjekt können jeweils ein Stift oder ein Röhrchen oder eine Kugel gewählt werden. Als Material hat sich im Besonderen Stahl oder Titan als geeignet erwiesen, da somit die Dimensionsstabilität und Röntgenstrahlen absorbierende Fähigkeit besonders günstig sind. Wird eine Kugel als Referenzobjekt gewählt, so hat es sich als günstig erwiesen, deren Durchmesser bei 0,5 mm zu wählen.

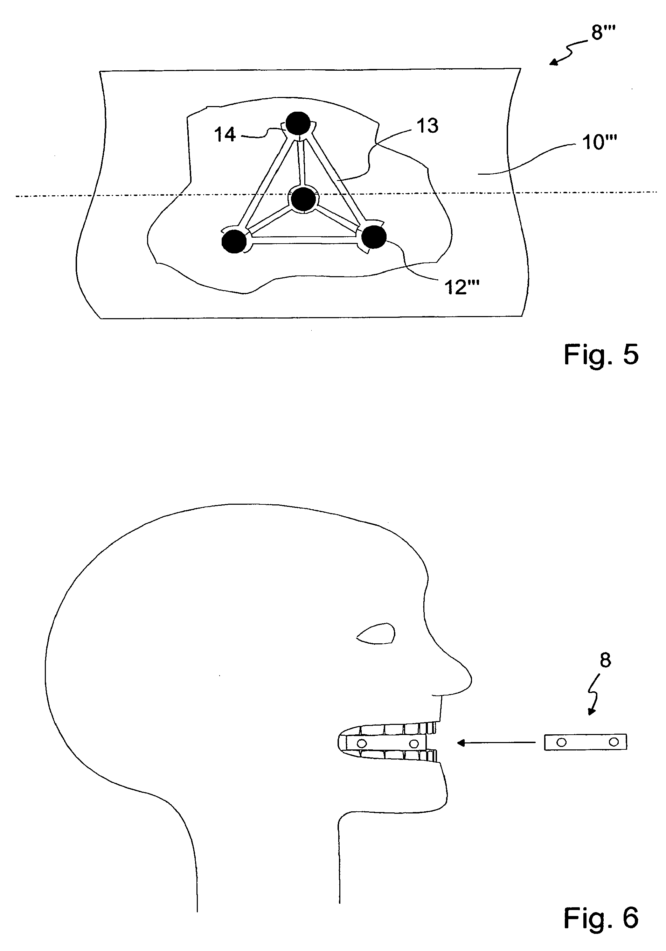

Mit Fig. 5 wird ein räumlicher Verbund mit mehr als zwei Referenzobjekten 12"', die in den schematisch dargestellten Hauptkörper 10"' eingebracht sind, dargestellt. Indem mehr als zwei Referenzobjekte 12'" im räumlichen Verbund zur Verfügung stehen, können weitere Informationen über Lage und Ausrichtung zum Untersuchungsobjekt 2 gewonnen werden, die der Auswerteeinheit 6 vom Aufnahmemedium 4 zur Verfügung gestellt werden, so dass am Bildschirm 5 durch individuelle Auswahl über das Bedienfeld 7 noch detaillierter und weiter in höherem Maße größenordnungsgerechte Abbildungen des Untersuchungsobjektes, z. B. in mehreren Ebenen, zur Anzeige bzw. zur weiteren Speicherung gebracht werden können. Die Verbindungsstege 13 zwischen den Referenzobjekten 12"', können aus nahezu röntgenstrahlen-durchlässigem Material bestehen und z. B. aus Kunststoff gebildet sein. Zur Aufnahme der Referenzobjekte weisen die Verbindungsstege Fortsätze 14 auf, die die Referenzobjekte geeignet aufnehmen können.

Mit Fig. 6 wird eine gewöhnliche Einbringung des erfindungsgemäßen dentalmedizinischen Erzeugnisses in den Mundraum des Patienten und dessen Fixierung durch Aufbeißen dargestellt. Das dentalmedizinische Erzeugnis 8 wird in den Mundraum des Patienten eingeführt und vom Patienten durch Aufbeißen fixiert. Das Aufnahmemedium 4 kann, wie obig dargestellt, ein Halbleiterarray sein. Als Aufnahmemedium 4 kommt jedoch auch ein herkömmlicher Röntgenfilm infrage, der dem Patienten zusammen mit dem dentalmedizinischen Erzeugnis 8 in den Mund gegeben wird. Dann ist es erforderlich, dass der Röntgenfilm jeweils ausgelesen wird, z.B. über eine Kamera, und das dann vom Untersuchungsobjekt und von mindestens einem Referenzobjekt 12 erhaltene digitale Bild einer der obigen Auswerteeinheit 6 entsprechenden Auswerteeinheit in Verbindung mit einem Bedienfeld 7 zugeführt wird, um jeweils die einzelne, auf dem Röntgenfilm vorliegende Aufnahme, auf einem obigem Bildschirm 5 entsprechenden Bildschirm größenordnungsgerecht ausgewertet zur Anzeige zu bringen.

### Bezugszeichenliste:

- 1: Röntgenstrahlungsquelle
- 1': Röntgenstrahlen
- 2: Untersuchungsobjekt
- 3: Referenzobjekt, allgemein
- 4: Aufnahmemedium
- 5: Bildschirm
- 6: Auswerteeinheit
- 7: Bedienfeld
- 8: Dentalmedizinisches Erzeugnis
- 10: Hauptkörper(10', 10", 10"')
- 11: Innenraum
- 12: Referenzobjekt (von Erfindung, 12', 12", 12")
- 13: Verbindungsstege
- 14: Fortsätze

## Patentansprüche

1. Dentalmedizinisches Erzeugnis (8) zur größenordnungsgerechten Auswertung von Röntgenaufnahmen von zu diagnostizierenden Bereichen (2), insbesondere Zahn- und/oder Kieferknochen-Bereichen, innerhalb der Mundhöhle eines Patienten, aufweisend einen Hauptkörper (10), dessen Gestalt so ist, dass er vom Patienten aufnehmbar ist und in unmittelbarer Nähe des durch Röntgen zu diagnostizierenden Bereiches (2) fixierbar ist, bestehend aus einem röntgenstrahlen-durchlässigem Material, wobei mindestens ein formstabiles Referenzobjekt (12) aus röntgenstrahlen-absorbierendem Material besteht, wobei das Referenzobjekt (12) im Innenraum des Hauptkörpers (10) aufgenommen ist, wobei der Hauptkörper (10) kugelförmig ist oder eine längliche Gestalt mit einem im Wesentlichen kreisrunden Querschnitt aufweist, und das Material des Hauptkörpers (10) nachgiebig bzw. weich ist,
**dadurch gekennzeichnet, dass**
der Hauptkörper (10) durch eine Zellstoff- bzw. Watterolle gebildet ist.

2. Dentalmedizinisches Erzeugnis nach Anspruch 1, **dadurch gekennzeichnet, dass** das mindestens eine Referenzobjekt (12) ein Stift oder ein Röhrchen oder eine Kugel ist.

3. Dentalmedizinisches Erzeugnis nach Anspruch 2, **dadurch gekennzeichnet, dass** das Material des mindestens einen Referenzobjektes (12) Stahl oder Titan ist.

4. Dentalmedizinisches Erzeugnis nach mindestens einem der Ansprüche 1 bis 3, **dadurch gekennzeichnet, dass** eine Kugel als Referenzobjekt (12, 12') vorgesehen ist.

5. Dentalmedizinisches Erzeugnis nach mindestens einem der Ansprüche 1 bis 3, **dadurch gekennzeichnet, dass** zwei Kugeln als Referenzobjekte (12") vorgesehen sind.

6. Dentalmedizinisches Erzeugnis nach Anspruch 4 oder 5, **dadurch gekennzeichnet, dass** der Durchmesser einer Kugel z. B. 0,5 mm betragt.

7. Dentalmedizinisches Erzeugnis nach mindestens einem der Ansprüche 1 bis 3, dass mehr als zwei Referenzobjekte (12"') vorgesehen sind, die einen räumlichen Verbund bilden, wobei die Verbindungsstege (13) zwischen den Referenzobjekten aus nahezu röntgenstrahlen-durchlässigem Material bestehen.

8. Verwendung eines dentalmedizinischen Erzeugnisses nach einem der Ansprüche 1 bis 7,
**dadurch gekennzeichnet, dass**
für die größenordnungsgerechte Auswertung von Röntgenaufnahmen von zu diagnostizierenden Bereichen, insbesondere von Zahn- und / oder Kieferknochen-Bereichen, innerhalb der Mundhöhle eines Patienten, mindestens ein dentalmedizinisches Erzeugnis (8) dem Patienten in den Mund gegeben wird und in der Weise in unmittelbarer Nähe des zu diagnostizierenden Bereiches fixierbar ist, indem der Patient das mindestens eine dentalmedizinische Erzeugnis durch Aufbeißen fixiert und ein beim Röntgen zunächst verzerrtes Abbild des diagnostizierten Bereiches über die bekannte Größe des mindestens einen im Innenraum eines Hauptkörpers des dentalmedizinischen Erzeugnisses aufgenommenen Referenzobjektes aus röntgenstrahlen-absorbierendem Material korrigierend ausgerechnet wird, so dass ein größenordnungsgerechtes Bild bzw. eine größenordnungsgerechte Anzeige entsteht, bzw. die Dimensionen der Knochenstruktur im Diagnosebereich unter Verwendung des Referenzobjektes berechenbar ist.

## Claims

1. A dental product (8) for magnitude-correct evaluation of x-ray images of areas to be diagnosed (2), particularly tooth areas and/or jaw bone areas, within the oral cavity of a patient, having a main body (10), which is configured such that it can be taken in by the patient and can be fixed in direct proximity to the area that is to be diagnosed by x-ray (2), the main body comprising a radiotransparent material, wherein at least one dimensionally stable reference object (12) is present, wherein the reference (12 object is received in the interior of the main body (10), wherein the main body (10) is spherical or elongated in shape with a substantially circular cross section, and the material of the main body (10) is soft and pliable, **characterized in that** the main body (10) is formed by a cellulose or cotton roll.

2. The dental product according to claim 1, **characterized in that** the at least one reference object (12) is a rod or a small tube or a sphere.

3. The dental product according to claim 2, **characterized in that** the material of the at least one reference object (12) is steel or titanium.

4. The dental product according to at least one of claims 1 to 3, **characterized in that** a sphere is provided as the reference object (12, 12').

5. The dental product according to at least one of claims 1 to 3, **characterized in that** two spheres are provided as reference objects (12").

6. The dental product according to claims 4 or 5, **characterized in that** the diameter of a sphere is, for example, 0.5 mm.

7. The dental product according to at least one of claims 1 to 3, **characterized in that** more than two reference objects (12"') are provided, which form a three-dimensional composite, wherein the connecting webs (13) between the reference objects comprise an almost radiotransparent material.

8. The use of a dental product according to any one of claims 1 to 7, **characterized in that** for the magnitude-correct evaluation of x-rays of areas to be diagnosed, particularly tooth areas and/or jaw bone areas, within the oral cavity of a patient, at least one dental product (8) is administered in the mouth of a patient and in this way is fixed in direct proximity to the area to be diagnosed, **in that** the patient fixes the at least one dental product through biting and an image of the diagnostic area which is initially distorted during x-ray is correctively calculated by means of the known size of the at least one reference object made from x-ray absorbing material which is taken into the interior of a main body of the dental product, so that a magnitude-correct image or a magnitude-correct display results, and the dimensions of the bone structure in the diagnostic area can be calculated through the use of the reference object.

## Revendications

1. Produit (8) à usage dentaire et médical servant à évaluer en ordre de grandeur des radiographies de régions (2) à diagnostiquer, en particulier de régions dentaires et/ou maxillaires dans la cavité buccale d'un patient, comprenant un corps principal (10) dont la forme est telle qu'il peut être pris par le patient et fixé à proximité immédiate de la région (2) à diagnostiquer par rayons X, et qui est constitué d'un matériau transparent aux rayons X, au moins un objet de référence (12) de forme stable étant constitué d'un matériau absorbant les rayons X, l'objet de référence (12) étant logé à l'intérieur du corps principal (10), le corps principal (10) étant sphérique ou ayant une forme allongée de section transversale sensiblement circulaire, et le matériau du corps principal (10) étant élastique ou souple,
**caractérisé en ce que**
le corps principal (10) est formé par un rouleau de pâte à papier ou de ouate.

2. Produit à usage dentaire et médical selon la revendication 1, **caractérisé en ce que** l'au moins objet de référence (12) est une tige ou un tubule ou une bille.

3. Produit à usage dentaire et médical selon la revendication 2, **caractérisé en ce que** le matériau de l'au moins objet de référence (12) est en acier ou en titane.

4. Produit à usage dentaire et médical selon l'une au moins des revendications 1 à 3, **caractérisé en ce qu'**une bille est prévue comme objet de référence (12, 12').

5. Produit à usage dentaire et médical selon l'une au moins des revendications 1 à 3, **caractérisé en ce que** deux billes sont prévues comme objets de référence (12").

6. Produit à usage dentaire et médical selon la revendication 4 ou la revendication 5, **caractérisé en ce que** le diamètre d'une bille est par exemple de 0,5 mm.

7. Produit à usage dentaire et médical selon l'une au moins des revendications 1 à 3, **caractérisé en ce qu'**il est prévu plus de deux objets de référence (12"') qui forment un composite tridimensionnel, les nervures de liaison (13) placées entre les objets de référence étant constituées d'un matériau à peu près transparent aux rayons X.

8. Utilisation d'un produit à usage dentaire et médical selon l'une quelconque des revendications 1 à 7, **caractérisé en ce que**, pour effectuer l'évaluation en ordre de grandeur de radiographies de régions à diagnostiquer, en particulier de régions dentaires et/ou maxillaires, à l'intérieur de la cavité buccale d'un patient, au moins un produit à usage dentaire et médical (8) est placé dans la bouche du patient et peut être fixé à proximité immédiate de la région à diagnostiquer de manière à ce que le patient fixe l'au moins un produit à usage dentaire et médical en le mordant, et une représentation, tout d'abord déformée lors de la radiographie, de la région à diagnostiquer est calculé avec corrections par référence à la taille connue de l'au moins un objet de référence en matériau absorbant les rayons X qui est placé à l'intérieur d'un corps principal du produit à usage dentaire et médical de façon à générer une image en ordre de grandeur ou une représentation en ordre de grandeur ou à pouvoir calculer les dimensions de la structure osseuse dans la région du diagnostic à l'aide de l'objet de référence.
